# EUROPEAN PATENT APPLICATION

(11) **EP 1 953 247 A1**
(43) Date of publication of application: **06.08.2008**
(21) Application number: 08101034.0
(22) Date of filing: 29.01.2008
(51) Int. Cl.: C12Q 1/68

(54) **A method for measuring the number of oral streptococci, and a PCR primers-probe set used for the same**

(30) Priority: 31.01.2007 JP 2007021423
(71) Applicant: GC Corporation, Itabashi-ku Tokyo 174-8585 (JP)
(72) Inventor: Matsumoto, Yuko, Tokyo, Tokyo 174-8585 (JP)
(74) Representative: Cohausz & Florack

(57) **Abstract**

There is provided a real time PCR assay capable of calculating a ratio of the number of mutans streptococci to the number of oral streptococci present in an oral cavity, in a short time.

The object can be solved by a method for measuring the number of oral streptococci using the combination of a forward primer comprising a primer oligonucleotide part of at least 15 sequential bases in the base sequence of SEQ ID NO: 1 and a reverse primer comprising a probe oligonucleotide part of at least 15 sequential bases in the base sequence of SEQ ID NO: 2; and a probe for measuring the number of oral streptococci, comprising a nucleotide part consisting of at least 10 sequential bases in the base sequence of SEQ ID NO: 3 or 4.

## Description

### TECHNICAL FIELD

The present invention relates to a method for measuring the number of oral streptococci, and a PCR primers-probe set used for the same.

### BACKGROUND ART

It is known that the presence of mutans streptococci in the oral cavity of a human is closely associated with a development of dental caries. Of the mutans streptococci in the oral cavity of a human, Streptococcus mutans and Streptococcus sobrinus are considered the main bacterial cause of dental caries.

Recently it has become known that a ratio of the number of mutans streptococci to the number of all kinds of oral streptococci in the oral cavity is an important part of the risk of a development of dental caries, in addition to the presence of mutans streptococci in the oral cavity. In this connection, it has been reported that a human with a high ratio of the number of mutans streptococci to the number of all kinds of oral streptococci present in an oral cavity, is susceptible to the development of dental caries (non-patent reference 1, non-patent reference 2, and non-patent reference 3). For a calculation of the ratio, it is important that the number of mutans streptococci and the number of all oral streptococci including the mutans streptococci are accurately measured. Hitherto, the measurement of the number of mutans streptococci and the number of all oral streptococci including the mutans streptococci has been carried out by a method of culturing bacteria in the oral cavity. However, it takes several days to measure the ratio, because the culture and identification of streptococci is a lengthy process.

For the measurement of the number of mutans streptococci and the measurement of the number of oral streptococci, the method of measuring the number of mutans streptococci, i.e. Streptococcus mutans or Streptococcus sobrinus, by means of a real time PCR assay using specific primers and a probe designed for each strain, has been developed, and therefore the number of mutans streptococci in saliva or bacterial plaque can be measured in a short time by this real time PCR assay (non-patent reference 4).

For the measurement of the number of oral streptococci including Streptococcus salivarius, Streptococcus sanguis, Streptococcus mitis, Streptococcus oralis, and Streptococcus gordonii, which are not associated with the development of dental caries, and mutans streptococci, a real time PCR assay capable of measuring oral streptococci present in an oral cavity has been not developed, and thus a culture method, which is a lengthy process, is used. Accordingly, it takes several days in total to measure the ratio of the number of mutans streptococci to the number of all oral streptococci.

Under these circumstances, a method for a detection of the risk of development of dental caries is very time-consuming, and thus is not efficient. Therefore, this method was not suitable for the detection of the risk of development of dental caries, as a method for testing many samples in a medical laboratory and so on. This is because the number of oral streptococci in an oral cavity is measured by a culture method, whereby the ratio of the number of mutans streptococci thereto is calculated.

### [Non-Patent Reference No. 1]

"Shoki ushokuno maneijimento (Management of early caries)" Quintessence Publishing CO., Ltd, August 10, 2004, p.29-31, and p.124-125
[Non-Patent Reference No. 2] Archives of Oral Biology, England, 1996, vo141, p.167-73
[Non-Patent Reference No. 3] Japanese Journal of Bacteriology, 2001, vol.56, p.334
[Non-Patent Reference No. 4] Journal of Clinical Microbiology, U.S.A, 2003, vol.41, p.4438-41

### DISCLOSURE OF THE INVENTION

### PROBLEMS TO BE SOLVED BY THE INVENTION

In order to measure the ratio of the number of mutans streptococci to the number of oral streptococci in a short time, the present inventors conducted intensive studies into a method for accurately measuring the number of oral streptococci by real time PCR assay. As a result, the inventors focused their attention on the base sequence of 16S ribosomal RNA included in 30S subunit of prokaryotic ribosome, and designed primers, and primers and probes for use in real time PCR assay by selecting regions of base sequences commonly present in all oral streptococci. Then, the inventors carried out real time PCR assays using the primers, and the primers and probes, and found the specific primers and probes for measuring the number of oral streptococci present in an oral cavity. The number of oral streptococci obtained by the real time PCR assay using the specific primers and probes accurately correlates with those obtained by the culture method.
The present invention is based on the above findings.

### MEANS FOR SOLVING THE PROBLEMS

The present invention relates to a primer set for measuring the number of oral streptococci, comprising at least one forward primer comprising an oligonucleotide part of at least 15 sequential bases in a base sequence of SEQ ID NO: 1, and at least one reverse primer comprising an oligonucleotide part of at least 15 sequential bases in a base sequence of SEQ ID NO: 2.
According to a preferable embodiment of the primer set of the present invention, the forward primer is an oligonucleotide of the base sequence of SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, or SEQ ID NO:14, or a mixture of two or more the oligonucleotides, and the reverse primer is an oligonucleotide of the base sequence of SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, or SEQ ID NO:26, or a mixture of two or more the oligonucleotides.
Further, the present invention relates to a primers-probe set comprising the primer set described above, and at least one probe comprising an oligonucleotide part of at least 10 sequential bases in a base sequence of SEQ ID NO: 3 or 4.
According to a preferable embodiment of the primers-probe set of the present invention, the probe is an oligonucleotide of the base sequence of SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, or SEQ ID NO:45 or a mixture of two or more of these oligonucleotides.
Further, the present invention relates to a method for measuring the number of oral streptococci by a real time PCR assay using the primer set described above. Furthermore, the present invention also relates to a method for measuring the number of oral streptococci by a real time PCR assay using the primers-probe set described above.
Still further, the present invention relates to a method for measuring the number of oral streptococci by a real time PCR assay using the primers-probe set described above, or a real time PCR kit for measuring the number of oral streptococci, comprising the primer set described above.
The term measurement or measuring as used herein means quantity or quantifying. However, the oral streptococci are detected by quantifying the oral streptococci. Therefore, it is not excluded that the primer-probe sets, and the method for measuring the number of oral streptococci of the present invention are used in qualitative detection of streptococci. EFFECTS OF THE PRESENT INVENTION

According to the present invention, the number of oral streptococci can be measured accurately in a short time. The risk of development of dental caries can be effectively detected by calculating the ratio of the number of mutans streptococci to the number of oral streptococci. Further, many samples can be measured, compared with the culture method, and thus the measuring method of the present invention can be carried out effectively in a medical laboratory and so on.

### BEST MODE FOR CARRYING OUT THE INVENTION

The primer set, and the primers-probe set can be used for quantifying the number of oral streptococci. The base sequence of primers and probes in the primer set or the primers-probe set are based on the base sequence of the 16S ribosomal RNA gene, which is included in 30S ribosome subunit of streptococci.
The term oral streptococci as used herein means all kinds of oral streptococci present in an oral cavity. The oral streptococci includes, for example, Streptococcus salivarius, Streptococcus sanguis, Streptococcus mitis, Streptococcus oralis, Streptococcus gordonii, and mutans streptococci, which includes Streptococcus mutans and Streptococcus sobrinus.
The present inventors selected the identical base sequence regions in the 16S ribosomal RNA gene of Streptococcus salivarius, Streptococcus sanguis, Streptococcus mitis, Streptococcus oralis, Streptococcus gordonii, and mutans streptococci, which includes Streptococcus mutans and Streptococcus sobrinus, and designed primers and probes. However, in the case of merely selecting primers and probes having identical base sequence regions in all oral streptococci without careful study, the measurement value obtained by PCR could not be correlated with that obtained by the culture method. Therefore, the present inventors selected the most appropriate regions base sequence for the measurement of streptococci and are designed as the primer set, and the primers-probe set, whereby developing the measuring method capable of obtaining the measurement value correlated with the number of oral streptococci obtained by the culture method.

The primer in the primer set of the present invention comprises a primer oligonucleotide part of at least 15 sequential bases in the base sequence of SEQ ID NO: 1, SEQ ID NO: 2, SEQ ID NO: 3, and SEQ ID NO: 4(hereinafter referred to as a primer oligonucleotide part 1, a primer oligonucleotide part 2, a primer oligonucleotide part 3, and a primer oligonucleotide part 4, respectively). Each of the primers comprising each of the primer oligonucleotide parts is referred to as a region 1 primer, a region 2 primer, a region 3 primer, and a region 4 primer, respectively. A length of each primer oligonucleotide parts is preferably 15-mer or more, more preferably 16-mer or more, most preferably 18-mer or more.
Further, the probe in the primers-probe set of the present invention comprises a probe oligonucleotide part of at least 10 sequential bases in the base sequence of SEQ ID NO: 3, and SEQ ID NO: 4 (hereinafter referred to as a probe oligonucleotide part 3, a probe oligonucleotide part 4, respectively). Each of the probes comprising each of the probe oligonucleotide parts is referred to as a region 3 probe, and a region 4 probe, respectively. A length of each probe oligonucleotide parts is preferably 10-mer or more, more preferably 12-mer or more, most preferably 14-mer or more. The base sequencees are shown as follows.
Region 1(SEQ ID NO: 1):5`-GGTTTTCGGATCGTAAAGCTCTGTTGTAAG-3' Region 2(SEQ ID NO: 2):5`-AATCCGGACAACGCTCGGGACCTACGTA-3` Region 3(SEQ ID NO: 3):5`-TACCAGAAAGGGACGGCTAACTACGTGCCA-3` Region 4(SEQ ID NO: 4):5'-TGGCACGTAGTTAGCCGTCCCTTTCTGGTA-3'

The base sequence represented by SEQ ID NO: 46 is that of a single strand DNA of a double strand DNA of a 16S ribosomal RNA gene of Streptococcus mutans, which is one of the streptococci present in an oral cavity. The base sequence represented by SEQ ID NO: 1 corresponds to the base sequence of the 412th to 440th bases region in the base sequence of SEQ ID NO: 46. The base sequence represented by SEQ ID NO: 2 corresponds to the complementary base sequence of 530th to the 555th bases region in the base sequence of SEQ ID NO: 46. The base sequence represented by SEQ ID NO: 3 corresponds to the base sequence of the 487th to 516th bases region in the base sequence of SEQ ID NO: 46. The base sequence represented by SEQ ID NO: 4 corresponds to the complementary base sequence of the 487th to 516th bases region in the base sequence of SEQ ID NO: 46. The region 1 primer, the region 2 primer, the region 3 primer, and the region 4 primer, and the region 3 probe and the region 4 probe are selected from the nucleotide sequence of the 16S ribosomal RNA gene, whereby the measuring method capable of exactly measuring the number of oral streptococci present in an oral cavity is developed.

The primer may be an oligonucleotide consisting of at least 15 or more sequential bases in the base sequence of SEQ ID NO: 1, SEQ ID NO:2, SEQ ID NO: 3, or SEQ ID NO: 4, preferably an oligonucleotide consisting of at least 15 or more sequential bases in the base sequence of SEQ ID NO: 1, or SEQ ID NO:2, more preferably an oligonucleotide consisting of at least 15 or more sequential bases in 2nd to 30th bases of the base sequence of SEQ ID NO: 1, or in 3rd to 25th bases of the base sequence of SEQ ID NO:2. More particularly, the primer may be an oligonucleotide consisting of the base sequence any one of SEQ ID NO: 5 to 14 or any one of SEQ ID NO: 15 to 26. The primers of SEQ ID NO: 5 to 26 are shown in Table 1 and 2.

**Table 1**

| **Primer** | **SEQ ID** | **Base Sequence** |
|---|---|---|
| F1 | SEO ID 5 | 5'-GTTTTCGGAT CGTAAAGCTC TGTT-3' |
| F2 | SEO ID 6 | 5 '-TTTTCGGATC GTAAAGCTCT GTT-3' |
| F3 | SEO ID7 | 5 '-TTTTCGGATC GTAAAGCTCT GTTG-3' |
| F4 | SEO ID8 | 5 '-TTTCGGATCG TAAAGCTCTG TTG-3' |
| F5 | SEQtD9 | 5'-TTCGGATCGT AAAGCTCTGT TG-3' |
| F6 | SEQ ID 10 | 5 '-TTCGGATCGT AAAGCTCTGT TGT-3' |
| F7 | SEQ ID11 | 5'-TTCGGATCGT AAAGCTCTGT TGTA-3' |
| F8 | SEQ ID12 | 5 '-TCGGATCGTA AAGCTCTGTT GTAA-3' |
| F9 | SEQ ID13 | 5 '-TCGGATCGTA AAGCTCTGTT GTAAG-3' |
| F10 | SEQ ID 14 | 5'-CGGATCGTAA AGCTCTGTTG TAAG-3' |

**Table 2**

| **Primer** | **SEQ ID** | **Base Sequence** |
|---|---|---|
| R1 | SEQ ID 15 | 5'-TCCGGACAAC GCTCGG-3' |
| R2 | SEQ ID 16 | 5 '-CGGACAACGC TCGGGA-3' |
| R3 | SEQ ID 17 | 5 '-CGGACAACGC TCGGGAC-3' |
| R4 | SEQ ID 18 | 5 '-CGGACAACGC TCGGGACCTA-3' |
| R5 | SEQ ID 19 | 5 '-GGACAACGCT CGGGACC-3' |
| R6 | SEQ ID 20 | 5'-GGACAACGCT CGGGACCT-3' |
| R7 | SEQ ID 21 | 5 '-GGACAACGCT CGGGACCTA-3' |
| R8 | SEQ ID 22 | 5 '-GGACAACGCT CGGGACCTAC-3' |
| R9 | SEQ ID 23 | 5 '-ACAACGCTCG GGACCTACG-3' |
| R10 | SEQ ID 24 | 5 '-CAACGCTCGG GACCTACG-3' |
| R11 | SEQ ID 25 | 5 '-CAACGCTCGG GACCTACGT-3' |
| R12 | SEQ ID26 | 5 '-CAACGCTCGG GACCTACGTA-3' |

However, the primer in the present invention is not limited to the above primers, so long as the number of oral streptococci can be accurately measured by means of the primers. The primer may comprise the primer oligonucleotide part of at least 15 sequential bases in the base sequence of SEQ ID NO:1, SEQ ID NO:2, SEQ ID NO:3, or SEQ ID NO:4. In the primer comprising the primer oligonucleotide part, the primer may contain nucleotides in base sequences of a 16S ribosomal RNA gene other than the base sequence of the SEQ ID NO: 1 to 4, or other nucleotides consisting of an artificial base sequence. For example, 1 to 10 nucleotides can be added to a 5'-terminus of the above mentioned oligonucleotide. Further, a primer having an oligonucleotide sequence in which one to several nucleotides are substituted or 10% or less of nucleotides are mismatched in the above mentioned oligonucleotide, can be used.

. A length of the primer is not particularly limited, but is preferably 15-mer to 35-mer, more preferably 20-mer to 30-mer, most preferably 22-mer to 28-mer. Further, a length of the region 1 primer is not particularly limited, but is preferably 15-mer to 35-mer, more preferably 20-mer to 29-mer, most preferably 22-mer to 25-mer. Furthermore, a length of the region 2 primer is not particularly limited, but is preferably 15-mer to 35-mer, more preferably 16-mer to 25-mer, most preferably 16-mer to 20-mer.

The primer set of the present invention comprises a forward primer and a reverse primer. As the forward primer in the primer set, a primer or a combination of two or more primers can be used. Further, as the reverse primer in the primer set, a primer or a combination of two or more primers can be used. The term forward primer as used herein means a forward sided primer in the single stranded nucleotide of the base sequence of SEQ ID NO: 46, and the term reverse primer as used herein means a reverse sided primer in the single stranded nucleotide of the base sequence of SEQ ID NO: 46. More particularly, the forward primer is the region 1 primer or the region 3 primer, and the reverse primer is the region 2 primer or the region 4 primer. A combination of a primer set is not limited, so long as it is a combination of the forward primer and the reverse primer. As the primer set, there may be mentioned a combination of the region 1 primer and the region 2 primer, a combination of the region 1 primer and the region 4 primer, and a combination of the region 3 primer and the region 2 primer. The combination of the region 1 primer and the region 2 primer is preferable.

A primers-probe set of the present invention, comprises the primer set and at least one probe, and more specifically, comprises at least one region 1 primer (as a forward primer), at least one region 2 primer (as a reverse primer) and at least one probe. As the probe in the primers-probe set, a probe or a combination of two or more probes can be used. Further, as the forward primer in the primers-probe set, a primer or a combination of two or more primers can be used. Furthermore, as the reverse primer in the primers-probe set, a primer or a combination of two or more primers can be used. The probe is an oligonucleotide for detecting genomic DNA, cDNA or the like by means of the method using a hybridization technique, preferably for use in real time PCR assay, particularly, the TaqMan method. The probe can be designed from the base sequence region between the region 1 primer and the region 2 primer in the base sequence of SEQ ID NO: 46. As the probe, there may be mentioned a nucleotide which may hybridized to the nucleotide consisting of the base sequence of SEQ ID NO: 46, a nucleotide, which may hybridized to the complementary nucleotide thereof, or a mixture of these nucleotides. The probe which may hybridize to the nucleotide consisting of the base sequence of SEQ ID NO: 46 may be preferably the region 4 probe. The probe which may hybridize to the complementary nucleotide consisting of the base sequence of SEQ ID NO: 46 may be preferably the region 3 probe.

The probe is preferably an oligonucleotide of at least 10 or more sequential bases in the base sequence of SEQ ID NO: 3 or 4, more preferably an oligonucleotide of a base sequence of any one of SEQ ID NO: 27 to 45.

**Table 3**

| **Probe** | **SEQ ID** | **Base Sequence** |
|---|---|---|
| P1 | SEQ ID 27 | 5'-TACCAGAAAG GGACGGCT-3' |
| P2 | SEQ ID 28 | 5'-TACCAGAAAG GGACGGCTA-3' |
| P3 | SEQ ID 29 | 5 `-TACCAGAAAG GGACGGCTAA CTACGTGCG-3' |
| P4 | SEQ ID 30 | 5 '-ACCAGAAAGG GACGGCT-3' |
| P5 | SEQ ID 31 | 5 `-ACCAGAAAGG GACGGCTA-3' |
| P6 | SEQ ID 32 | 5'-ACCAGAAAGG GACGGCTAAC TACGTGCC-3' |
| P7 | SEO ID 33 | 5 '-CCAGAAAGGG ACGGCT-3' |
| P8 | SEQ ID 34 | 5 '-CCAGAAAGGG ACGGCTA-3' |
| P9 | SEQ ID 35 | 5 '-CCAGAAAGGG ACGGCTAACT ACGTGCC-3' |
| P10 | SEQ ID36 | 5 '-CAGAAAGGGA CGGCTAA-3' |
| P11 | SEO ID 37 | 5 '-CAGAAAGGGA CGGCTAACTA CGTGCCA-3' |
| P12 | SEQ ID 38 | 5 '-AGAAAGGGAC GGCTAAGTA-3' |
| P13 | SEQ ID 39 | 5'-AAAGGGACGG CTAACT-3' |
| P14 | SEQ ID 40 | 5'-AAAGGGACGG CTAACTA-3' |
| P15 | SEQ ID 41 | 5'-TAGCCGTCCC TTTCTGGTA-3' |
| P16 | SEQ ID 42 | 5'-AGCCGTCCCT TTCT-3' |
| P17 | SEQ ID 43 | 5 '-AGCCGTCCCT TTCTGGTA-3' |
| P18 | SEQ ID 44 | 5 '-GCCGTCCCTT TCT-3' |
| P19 | SEQ ID 45 | 5 '-CCGTCGCTTT CTGG-3' |

However, the probe in the present invention is not limited to the above probes, so long as the number of oral streptococci can be accurately measured by means of the probes. Therefore, a probe having an oligonucleotide in which one to several nucleotides are substituted or 10% or less of nucleotides are mismatched in the above mentioned oligonucleotide, can be used as a probe. The term hybridize as used herein is the same as the term hybridize used under conventional conditions of a real time PCR assay.

The probe used in a TaqMan assay is labeled by a reporter dye and a quencher dye. A labeled oligonucleotide, wherein a reporter dye is bound to one terminus such as a 5'-terminus thereof and a quencher dye is bound to the other terminus such as a 3'-terminus thereof, may be used as a probe. A conventional dye used in a PCR assay may be used as a dye without limitation. There may be mentioned 6-carboxy-fluorescein(FAM), tetrachloro-6-carboxyfluorescein(TET), 2,7-dimethoxy-4,5-ichloro-6-carboxyfluorescein(JOE), hexochloro-6-carboxyfluorescein(HEX) or the like, as a reporter dye. There may be mentioned, for example, 6-carboxy-tetramethylrhodamine(TAMRA), black hole quencher (BHQ) without native fluorescence or the like, as a quencher dye.

A length of the probe is not particularly limited, but is preferably 12-mer to 30-mer, more preferably 13-mer to 29-mer, most preferably 14-mer to 18-mer.

In the method for measuring the number of oral streptococci of the present invention, a polymerase chain reaction (PCR) may be used, and particularly, a real time PCR assay may be preferably used. As the real time PCR assay, there may be mentioned an intercalator-based real time PCR assay wherein an intercalator which can generate fluorescence by binding to double stranded DNA, such as SYBR Green I and the primer set, are added to a PCR reaction, and a TaqMan assay wherein the probe which is labeled with reporter dye of a 5'-terminus and a quencher dye of a 3'-terminus (hereinafter referred to as a TaqMan probe) and the primer set, are added to a PCR reaction. The real time PCR assay per se is well-known, and kits and devices therefor are commercially available. Therefore, the real time PCR assay can be easily carried out by only synthesizing the primer set, or the primers-probe set.

When the method for measuring the number of oral streptococci is carried out by an intercalator-based real time PCR assay, a primer set of a combination of the region 1 primer and the region 2 primer, the region 1 primer and the region 4 primer, or the region 3 primer and the region 2 primer, can be used, and particularly, a primer set of a combination of the region 1 primer and the region 2 primer is preferably used. When the method for measuring the number of oral streptococci is carried out by a TaqMan assay, the primer set of the region 1 primer as a forward primer and the region 2 primer as a reverse primer and the region 4 probe or the region 3 probe can be used. If either of the intercalator-based real time PCR assay or the TaqMan assay is used, the number of oral streptococci can be measured accurately. In particular, the TaqMan assay is preferable. In the TaqMan assay, a desired region of 16S ribosomal RNA gene of streptococci is specifically amplified for measurement, because a specific probe is further used in addition to two specific primers. Thus, the number of oral streptococci can be measured more accurately.

Conventionally performed PCR methods may be used in the present invention. In the PCR method, more specifically, a DNA synthesis reaction consisting of following three steps is repeated.
(1) First, a double stranded DNA as a template is heated and denatured to become a single stranded DNA.
(2) Next, two primers form a double strand DNA structure with a complementary region of the single stranded DNA respectively by cooling a reaction mixture containing the excess primers complementary to each end of a desired DNA region to be amplified.
(3) In the above condition, if deoxyribonucleotide triphosphate as a substrate and Taq DNA polymerase are added to the reaction mixture, complementary DNA are synthesized from the primers by the Taq DNA polymerase.
In the case of an intercalator-based real time PCR assay, an intercalator such as SYBR Green I and the like is added to the reaction mixture, and then a fluorescence of intercalator bound to the synthesized double stranded DNA is measured per each cycle. Meanwhile, in the case of the TaqMan assay, the TaqMan probe is added to a reaction mixture, and then fluorescence generated from a reporter dye detached from the degraded TaqMan probe is measured per each cycle.

As mentioned above, in the TaqMan method, a labeled probe, wherein a reporter dye is bound to one terminus such as a 5'-terminus of an ologonucleotide and a quencher dye is bound to the other terminus such as a 3'-terminus thereof, may be used. The reporter dye is a compound capable of generating fluorescence by a radiation of excitation light. If the reporter dye and quencher dye are bound together to one oligonucleotide, an energy absorbed by the reporter dye is absorbed into the quencher dye by an energy-transfer due to a close distance therebetween. Therefore, the reporter dye cannot be excited, and thus a natural fluorescence cannot be generated. This phenomenon is referred to as an optical quenching of fluorescence i.e. quenching. When the quenched probe is added to a reaction mixture, the probe hybridizes with a single strand DNA as a template in an annealing step. Subsequently, a Taq DNA polymerase synthesizes a new complementary DNA from a 3'-terminus of a primer and degrades the hybridized probe during synthesizing. Then, as a nick-translation occurs, the adjoining reporter dye and quencher dye are segregated, and thus the reporter dye subjected to an inhibition by quenching can generate a fluorescence. The above reaction occurs once per one molecule in a cycle of the PCR assay, and thus, an increase of a fluorescence intensity of the reporter dye almost correlates to the PCR reaction i.e. an amount of PCR products. According to the measuring method using the reporter dye and quencher dye, the measurement can be carried out without separating reaction products after the PCR assay, that is, can be carried out in real time.

As a sample to be measured according to the present invention, saliva, bacterial plaque or the like collected from an oral cavity may be used. The saliva or the bacterial plaque can be adjusted to an appropriate viscosity for measurement, if necessary. For example, after a subject to be tested has bitten paraffin wax, the saliva may be collected from the subject. Further, for example, the bacterial plaque may be collected from the subject by means of an exploratory needle or a pick.

In particular, the method for measuring the number of oral streptococci of the present invention comprises the following steps;
(1) a step of extracting DNA from samples to be tested,
(2) a step of amplifying the extracted DNA as a template by means of the primer set or the primers-probe set, and
(3) a step of detecting the amplified DNA.

The extraction of DNA contained in saliva or bacterial plaque collected from the oral cavity in the step 1, can be carried out by means of commercially available DNA extraction kit in accordance with an attached protocol. Alternatively, the saliva, bacterial plaque or the like is suspended in a buffer or a solution containing a detergent and the like for bacteriolysis, and the suspension is heated at 80 °C to 100 °C, for 1 to 30 minutes to lyse streptococci. The whole suspension or a supernatant after centrifuging the whole suspension can be used as the DNA sample.
In the DNA amplifying step in step (2), for example the TaqMan assay comprises the following reactions:
(a) If streptococci are present in a sample, an exponential DNA amplification occurs using a DNA of streptococci as a template DNA.
(b) During amplification, a TaqMan probe hybridizes to a template DNA. When a complementary DNA is synthesized using the template DNA hybridized with a primer, the TaqMan probe is degraded by Taq DNA polymerase with an exonuclease activity.
(c) Therefore, a reporter dye and a quencher dye, which are bound to the TaqMan probe, are segregated, whereby the reporter dye becomes detectable. In other words, if streptococci exists in the sample, the reporter dye generates a fluorescence and can be detected.
(d) The fluorescence intensity is dependent on the number of cycle of the PCR, and thus is increased exponentially.
(e) Several standard samples containing bacteria of a predetermined concentration are prepared, and a threshold cycle value (hereinafter referred to as Ct value) of each standard sample, at which fluorescence intensity is quickly increased, is determined. Then, a linear standard curve is obtained by plotting the Ct value on a longitudinal axis and a log of the predetermined concentration of standard samples on an abscissa axis.
(f) A Ct value of an unknown sample in a concentration of bacteria is examined. Then the resulting Ct value is applied to the above standard curve, whereby the bacteria concentration of the unknown sample is obtained.
In the detecting step of amplified DNA in step (3), the fluorescence intensity is measured per PCR cycle, whereby an increase of PCR products can be measured in real time.

A kit of the present invention can be used in the method for measuring the number of oral streptococci, and is characterized by comprising at least the forward primer and reverse primer. More preferably, the kit may comprise the primer set consisting of the region 1 primer and the region 2 primer, and the region 3 probe or the region 4 probe.

The kit of the present invention may contain the forward primer, reverse primer and probe, as a separate compound respectively, or as a mixture thereof. Further, the kit of the present invention may contain a desired reagent or an enzyme for real time PCR in addition to the primers and the probe.
The risk of development of dental caries can be predicted by determining the ratio of the number of mutans streptococci to the number of the oral streptococci. The number of the oral streptococci and the number of mutans streptococci may be measured by culturing a sample taken from an oral cavity. Further, the number of the oral streptococci and the number of mutans streptococci may be measured by means of a real time PCR assay using the sample. The number of the oral streptococci and the number of mutans streptococci obtained by the real time PCR assay preferably correlate with those obtained by the culture method.
The number of mutans streptococci, for example, may be measured by a real time PCR assay described in the after-mentioned Comparative Example 4. Further, the number of the oral streptococci may be measured by the real time PCR assay using the primers-probe set of the present invention. The ratio can be calculated by the number of mutans streptococci and the number of the oral streptococci obtained by the real time PCR assays, whereby the risk of a development of dental caries can be predicted.
More particularly, in order to determine the ratio, the number of mutans streptococci and the number of the oral streptococci in a sample taken from an oral cavity are measured by the real time PCR assays, and then the ratio of the number of mutans streptococci to the number of the oral streptococci is calculated.

### Function

The primers-probe set of the present invention, and some primers-probe sets used in after-mentioned comparative examples 1 to 3 are designed from regions selected from common base sequence regions present in 16S ribosomal RNA gene of Streptococcus salivarius, Streptococcus sanguis, Streptococcus mitis, Streptococcus oralis, Streptococcus gordonii, Streptococcus mutans, and Streptococcus sobrinus. According to the real time PCR assay using the primers-probe set of the present invention, the obtained measurement value can be correlated with the number of streptococci obtained by the culture method. However, according to the real time PCR assay by the primers-probe set used in comparative examples 1 to 3, the obtained measurement value cannot be correlated with that obtained by the culture method, or a favorable standard curve can not be prepared. The reason for this has not been fully elucidated, but is presumed to be as follows. However, the present invention is by no means limited to the following explanation.

In an oral cavity, several permanent bacteria are present together with streptococci, and it is considered that the kinds of the permanent bacteria other than streptococci and an amount thereof vary widely in individuals. A region of the base sequence for the primers-probe set of the present invention should be selected from a base sequence of a kind of certain streptococcus, but also from a common base sequence of all oral streptococci. Therefore, it is possible that a 16S ribosomal RNA gene of the permanent bacteria other than streptococci is detected, when a primers-probe set having a poor specificity is used. Further, as to the primers-probe set used in Comparative Example 3 described below, a linear standard curve was not obtained. In this case, a favorable PCR reaction could not occur. The reasons for this are presumed to be as follows. First, it is considered that the primer binding region of a desired DNA easily forms a secondary structure such as a hairpin loop when the desired DNA is denatured to become a single stranded DNA. In such cases, the binding of primers and probe to the desired DNA is inhibited. Second, it is considered that a primer dimmer is formed by a binding between primers. Third, it is considered that the primer or probe per se form a secondary structure such as a hairpin loop. Accordingly, to accurately measure the number of oral streptococci, the regions with a common base sequence of all oral streptococci are selected. Further, of the above regions, a region suitable for a real time PCR assay and usable to obtain an accurate measurement should be selected.

### Examples

The present invention now will be further illustrated by, but is by no means limited to, the following Examples.

### Extraction of DNA

0.2 mL of Saliva was collected from the oral cavity of five subjects to be tested, and was then suspended in 1.6 mL of sterilized water, to adjust the viscosity, and the whole was centrifuged to obtain a pellet. From the pellet, 0.2 mL of DNA sample was extracted using a DNA extraction kit (QuickGene DNA tissue kit S; FUJIFILM Corporation) in accodance with a manual attached to the kit.

### Example 1

The resulting DNA sample and the following amounts of reagents were mixed in a tube, to prepare a total volume of 20 µL of a reaction mixture

| | |
|---|---|
| TaqMan Fast Universal PCR Mater Mix : | 10 µL |
| 10µM of F6 foraward primer : | 0.4 µL (final concentration of 200nM) |
| 10µM of R8 reverse primer : | 0.4 µL(final concentration of 200nM) |
| 5µM of P8 probe : | 0.4 µL(final concentration of 100nM) |
| DNA sample : | 1.0 µL |
| sterilized water : | 7.8 µL |

The tubes containing 20 µL of the reaction mixture were placed in an Applied Biosystems 7500 Fast real time PCR system, and then a PCR reaction was carried out. After an incubation at 95°C for 20 seconds was carried out as a pretreatment, a cycle composed of treatments at 95°C for 3 seconds, and at 60°C for 30 seconds, was repeated 40 times. A fluorescence intensity was measured per each cycle. Chemical synthesized oligonucleotides were used as primers, and a chemical synthesized oligonucleotide, in which FAM is bound to a 5'-terminus thereof and BHQ is bound to a 3'-terminus thereof, was used as a probe.
In order to prepare a standard curve, a Streptococcus mitis ATCC49456 strain was cultured and diluted to prepare various concentrations of standard bacterial samples. A threshold cycle (Ct value) of each standard bacterial sample, at which fluorescence intensity is quickly increased, was examined, and then a standard curve wherein a longitudinal axis is Ct value, and an abscissa axis is a log of the concentration of standard bacterial samples is prepared. Simultaneously, Ct values of the samples were also examined and the bacterial concentration of the samples was estimated from a standard curve. The results are shown in Table 4.

### Example 2

The procedure described in Example 1 was repeated except that an F10 forward primer as a forward primer, an R4 reverse primer as a reverse primer, and a P13 probe as a probe were used. The results are shown in Table 4.

### Example 3

The procedure described in Example 1 was repeated except that an F3 forward primer as a forward primer, an R5 reverse primer as a reverse primer, and a P17 probe as a probe were used. The result are shown in Table 4.

### Example 4

The procedure described in Example 1 was repeated except that an F1 forward primer as a forward primer, an R2 reverse primer as a reverse primer, and a P19 probe as a probe were used. The results are shown in Table 4.

### Comparative Example 1

The procedure described in Example 1 was repeated except that an F11 forward primer (SEO ID NO:47) as a forward primer, an R13 reverse primer (SEQ ID NO:48) as a reverse primer, and a P20 probe (SEQ ID NO:49) as a probe were used. Each primer was designed on the basis of a common base sequence region in a 16S ribosomal RNA gene of Streptococcus salivarius, Streptococcus sanguis, Streptococcus mitis, Streptococcus oralis, and Streptococcus gordonii, streptococcus mutans and Streptococcus sobrinus. The base sequence of the F11 forward primer, R13 reverse primer, and P20 probe are shown as follows. The corresponding nucleotide numbers in a base sequence of Streptococcus mutans of SEQ ID NO:46 are shown in parentheses.
F11:5'-GTGTAGCGGTGAAATGCGTAGA-3' (678-699)
R13:5'-CCCGTCAATTCCTTTGAGTTTC-3' (902-923)
P20:5'-CAGGATTAGA1ACCCTGGTAG-3' (778-798)
The results are shown in Table 4.

### Comparative Example 2

The procedure described in Example 1 was repeated except that an F12 forward primer (SEQ ID NO: 50) as a forward primer, an R14 reverse primer (SEQ ID NO: 51) as a reverse primer, and a P21 probe (SEQ ID NO: 52) as a probe were used. Each primer was designed on the basis of a common base sequence region in a 16S ribosomal RNA gene of each oral Streptococci described in comparative Example 1. The base sequence of the F12 forward primer, R14 reverse primer, and P21 probe are shown as follows. The corresponding nucleotide numbers in a base sequence of Streptococcus mutans of SEQ ID NO:46 are shown in parentheses.
F12:5'-GGCTACACACGTGCTACAATGG-3*'* (1216-1237)
R14:5'-AAGGCCCGGGAACGTATTC-3*'* (1368-1386)
P21:5'-TCGCTAGTAATCGCG-3'(1336-1350) The results are shown in Table 4.
Unexpectedly, the real time PCR assay using the combination of the above primers and probe detected not only streptococci but also Porphyromonas gingivalis, Lactobacillus rhamnosus, Lactobacillus salivarius, and Lactobacillus casei.

### Comparative Example 3

The procedure described in Example 1 was repeated except that an F13 forward primer (SEQ ID NO: 53) as a forward primer, an R15 reverse primer (SEQ ID NO: 54) as a reverse primer, and a P22 probe (SEQ ID NO: 55) as a probe were used. Each primer was designed on the basis of a common base sequence region in a 16S ribosomal RNA gene of each oral Streptococci described in comparative Example 1. The base sequence of the F13 forward primer, R15 reverse primer, and a P22 probe are shown as follows. The corresponding nucleotide numbers in the base sequence of Streptococcus mutans of SEQ ID NO:46 are shown in parentheses.
F13:5`-CGCTAGTAATCGCGGATCAGCACG-3'(1337-1360)
R15:5`-TGTGACGGGCGGTGTGTA-3'(1388-1405)
P22:5`-CGGTGAATACGTTCCCGGGC-3'(1364-1383) The results are shown in Table 4.
Ten fold dilutions of DNA extract from cultured bacteria were amplified by the real time PCR assay using the combination of the above primers and probe, and an attempt to prepare a standard curve was made. In this case, however, a linear standard curve was not obtained, and thus the bacteria concentration could not be accurately measured.

### Comparative Example 4

DNA samples of saliva collected from the oral cavity of five subjects were measured by specific primers capable of detecting Streptococcus mutans, described in non-patent reference 4. The procedure described in Example 1 was repeated except that an Smut3368-F forward primer (SEQ ID NO: 56) as a forward primer, an Smut3481-R reverse primer (SEQ ID NO: 57) as a reverse primer, and an Smut3423T probe (SEQ ID NO: 58) as a probe were used. Each primer was designed on the basis of a gtfB gene of Streptococcus mutans. The base sequence of the Smut3368-F forward primer, Smut3481-R reverse primer, and Smut3423T probe are shown as follows.
Smut3368-F:5'-GCCTACAGCTCAGAGATGCTATTCT-3'
Smut3481-R:5'-GCCATACACCACTCATGAATTGA-3'
Smut3423T:5'-TGGAAATGACGGTCGCCGTTATGAA-3'
The results are shown in Table 4.
The saliva collected from the oral cavity of the subjects was diluted to an appropriate concentration, and inoculated on a Mitis Salivarius agar medium (hereinafter referred to as an MS agar medium) plate. The numbers of oral streptococci were measured by counting numbers of a colony. The results are shown in Table 4.

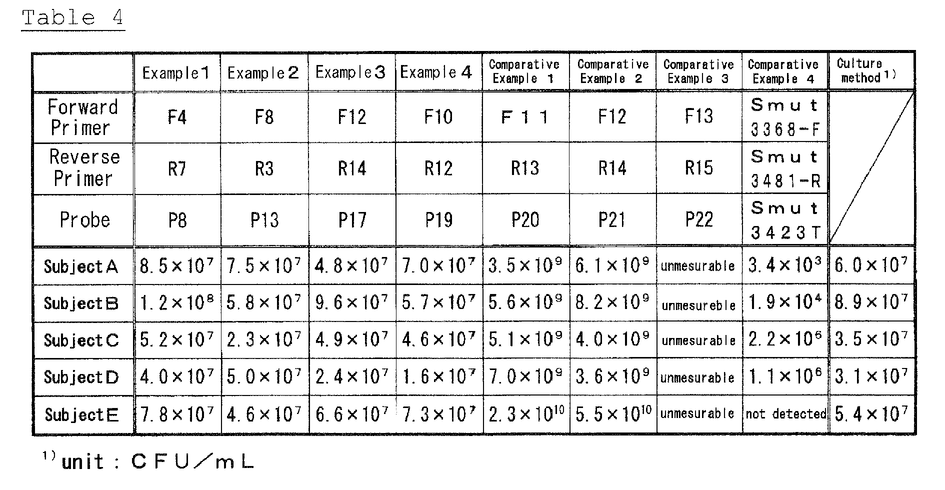

As shown in Table 4, according to the real time PCR assay using the primers-probe set of the present invention, the number of oral streptococci can be measured accurately in a short time. Further, the measurement value can be correlated with the number of streptococci obtained by the culture method. Therefore, the ratio of the number of mutans streptococci to the number of oral streptococci present in an oral cavity can be measured effectively. In fact, the ratio of the number of mutans streptococci to the number of oral streptococci could be calculated using the number of oral streptococci obtained in Examples 1 to 4, and the number of mutans streptococci i.e. Streptococcus mutans, obtained in Comparative Example 4. On the contrary, when the primers and probes described Comparative Examples 1 to 3 are used, the number of oral streptococci cannot be correlated with those obtained by a culture method. Therefore, the number of oral streptococci could not be accurately measured.

### Example 5

In Example 5, a real time PCR assay was carried out using typical streptococcus present in an oral cavity as a standard bacteria. It was confirmed that useful standard curves were obtained by the primers-probe set of the present invention in all oral streptococci strains.
The Streptococcus strains used here are shown as follows. The strain names used in each organization are shown in parentheses.
Streptococcus mutans(ATCC25175)
Streptococcus mutans(JCM5705)
Streptococcus mutans(OMZ)
Streptococcus mutans(JCM5706)
Streptococcus mutans(MT6229)
Streptococcus sobrinus (ATCC33478)
Streptococcus sobrinus (OMZ65)
Streptococcus sobrinus (6715)
Streptococcus sobrinus (MT8245)
Streptococcus mitis(ATCC903)
Streptococcus mitis (6249)
Streptococcus mitis(ATCC49456)
Streptococcus salivarius(JCM5707)
Streptococcus sanguis(ATCC10556)
Streptococcus sanguis(ATCC10557)
Streptococcus oralis(JCM12997)

Each strain was cultured in a Brain Heart Infusion (hereinafter referred to as BHI) medium and collected by centrifugation (5,000 rpm for 5 minutes). The resulting pellets were each suspended in a phosphate buffered saline (hereinafter referred to as PBS) to prepare a suspension of bacteria. DNA was extracted from each suspension using a DNA extarction kit [QuickGene DNA tissue kit S;FUJIFILM Corporation] in accordance with a manual attached to the kit. Simultaneously, each suspension of bacteria was diluted to an appropriate concentration, and inoculated on MS agar medium plates. The number of bacteria in the suspension was calculated by counting colony numbers.
The procedure described in Example 1 was repeated except that an F7 forward primer as a forward primer, an R7 reverse primer as a reverse primer, a P7 probe as a probe, and DNA extracted from each culture suspension as a DNA sample were used.
A threshold cycle (Ct value) of each DNA sample, at which a fluorescence intensity is quickly increased, was obtained, and then Ct values plotted on an abscissa axis, and a log of the bacterial concentration of each DNA samples plotted on a longitudinal axis, to prepare a standard curve.

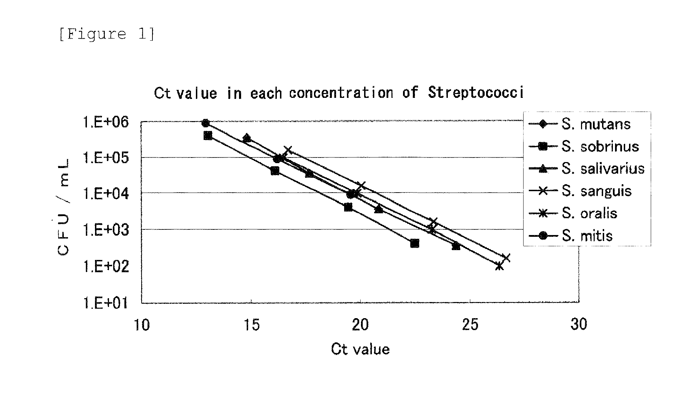

A graph plotted Ct values obtained by PCR and the number of bacteria in the original suspension is shown in Figure 1.

In Figure 1, S.mutans is the above ATCC25175 strain, S.sobrinus is the above ATCC33478 strain, S.mitis is the above ATCC49456 strain, and S.sanguis is the above ATCC10556 strain. Further, for S.mutans and S.sobrinus, several strains were examined. There was no difference of amplification among the strains. Therefore, the results revealed that the numbers of different streptococci can also be accurately measured according to the real time PCR assay.

### Example 6

Each streptococcus strain is examined to determine whether or not the number of bacteria obtained by the real time PCR assay correlates with that obtained by the culture method.
The bacteria strains described in Example 5 were cultured in a BHI liquid medium, and then the cultured bacteria were collected by centrifugation. Each of resulting pellets was suspended in PBS at an appropriate concentration, and then a DNA extract was prepared from the suspension. In addition, each of the above suspensions was inoculated on MS agar medium plate in parallel, and then the number of bacteria obtained by the culture method was calculated.
The procedure described in Example 1 was repeated except that an F8 forward primer as a forward primer, an R7 reverse primer and an R8 reverse primer as a reverse primer, a P8 probe as a probe, and DNA extracts obtained from each culture suspension were used as a DNA sample. The number of each bacteria was calculated from a standard curve obtained by Streptococcus mitis ATCC49456

The results of the real time PCR assay and the results of the culture method are shown in Table 5.

**Table 5**

| Species | Strain | Bacteria Numbers by Real time PCR | Bacteria Numbers by Culture method¹⁾ |
|---|---|---|---|
| Streptococcus mutans | ATCC25175 | 4.6 × 10⁸ | 5.5 × 10⁸ |
| Streptococcus mutans | JCM5705 | 6.3 × 10⁷ | 5.8 × 10⁷ |
| Streptococcus mutans | OMZ | 1.0 × 10⁸ | 1.1 × 10⁸ |
| Streptococcus mutans | JCM5706 | 9.8 × 10⁷ | 1.4 × 10⁸ |
| Streptococcus mutans | MT6229 | 8.0 × 10⁷ | 8.5 × 10⁷ |
| Streptococcus sobrinus | ATCC33478 | 3.5 × 10⁷ | 4.0 × 10⁷ |
| Streptococcus sobrinus | OMZ65 | 8.0 × 10⁸ | 9. 2 × 10⁸ |
| Streptococcus sobrinus | 6715 | 5.1 × 10⁹ | 4.4 × 10⁹ |
| Streptococcus sobrinus | MT8245 | 5.6 × 10⁹ | 4. 6 × 10⁹ |
| Streptococcus mitis | ATCC903 | 4.8 × 10⁸ | 5.2 × 10⁸ |
| Streptococcus mitis | 6249 | 1.4 × 10⁹ | 1.2 × 10⁹ |
| Streptococcus mitis | ATCC49456 | 2.2 × 10⁹ | 1.3 3 × 10⁹ |
| Streptococcus salivarius | JCM5707 | 7.3 × 10⁸ | 5.6 × 10⁸ |
| Streptococcus sanguis | ATCC10556 | 6.6 × 10⁷ | 4.9 × 10⁷ |
| Streptococcus sanguis | ATCC10557 | 1.9 × 10⁹ | 3.0 × 10⁹ |
| Streptococcus oralis | JCM12997 | 2.8 × 10⁸ | 4.0 × 10⁸ |

| | | | |
|---|---|---|---|
| ¹⁾ unit : CFU/mL | | | |

These results revealed that the number of bacteria obtained by the real time PCR assay closely correlated with that obtained by the culture method. Further, these results revealed that the number of bacteria can be accurately quantified by real time PCR assay in which plural primers were used as primer.

### Referential Example 1

In this Referential Example 1, it was confirmed that bacteria other than streptococci can not be detected by the real time PCR assay using the primers-probe set of the present invention.
The used bacteria strains in an oral cavity other than streptococci are shown as follows. The strain names used in each organization are shown in parentheses.
Lactobacillus salivarius(JCM1231)
Lactobacillus rhamnosus(JCM1563)
Lactobacillus casei (ATCC4646)
Actinobacillus actinomycetemcomitans (ATCC29524)
Porphyromonas gingivalis(ATCC33277)
Candida albicans(ATCC10231)
Candida krusei(JCM1609)
Actinomyces naeslundii(JCM8349)
Actinomyces odontolyticus (ATCC17929)

Each strain was cultured using the following medium, respectively. A composition of each media was a standard type, unless otherwise stated.
Lactobacillus genus: MRS medium
Actinobacillus actinomycetemcomitans : One liter of a liquid culture medium containing 37.5 g of BHI was sterilized by autoclave. The medium was allowed to stand in a mixed gas (N₂:H₂:CO₂=8:1:1) overnight, and then used.
Porphyromonas gingivalis: One liter of a liquid culture medium containing 37.5 g of BHI, 5 g of yeast extract, 5 g of tripticase peptone, 0.5 g of L-cysteine HCl and 1 mL of hemin-menadione solution, was sterilized by autoclave. The medium was allowed to stand in a mixed gas (N₂:H₂:CO₂=8:1:1) overnight, and then used. A Hemin-menadione solution was prepared as follows: 0.05 g of hemin was dissolved in 1 mL of 1M NaOH, and water was added to the solution up to 100 mL of total volume. The solution was sterilized by autoclave (Solution A). Then 0.2 g of menadione was dissolved in 20 mL of ethanol and then sterilized by autoclave (Solution B). One hundred mL of solution A and 1 mL of solution B were mixed to prepare a Hemin-menadione solution.
Candida genus: YM medium
Actinomyces genus: actinomyces medium(Becton, Dickinson and company)

The cultured bacteria were collected by centrifugation (5,000 rpm for 5 minutes), and resulting pellets were suspended in PBS to prepare suspensions of bacteria. DNA was extracted from each suspensions using an DNA extraction kit [QuickGene DNA tissue kit S;FUJIFILM Corporation] in accordance with a manual attached to the kit. Simultaneously, each suspension of bacteria was diluted to an appropriate concentration, and inoculated on MS agar medium plates. The number of bacteria in the suspension was calculated by counting colony numbers.
Lactobacillus genus: ROGOSA agar medium
Actinobacillus actinomycetemcomitans,Porphyromonas gingivalis: sheep blood agar (EIKEN Chemical Co., Ltd)
Candida genus: potato dextrose agar medium
Actinomyces genus: GAM semisolid medium
For the real time PCR assay, the procedure described in Example 1 was repeated. However, no amplification of PCR products was observed in any DNA sample obtained from the above cultured bacteria.

### INDUSTRIAL APPLICABILITY

According to the method for measuring the number of oral streptococci using the primers-probe set of the present invention, the ratio of the number of mutans streptococci to the number of oral streptococci can be calculated. The calculated ratio is usable for a detection of the risk of development of dental caries and useful for a prevention and treatment of dental caries. Further, according to the present invention, many samples can be measured, compared with the culture method, and thus the measurement can be carried out effectively in a medical laboratory and so on.

### BRIEF DESCRIPTION OF THE DRAWINGS

Fig. 1 is a graph showing Ct values of various oral streptococci obtained by the real time PCR assay and the numbers of streptococci contained in the original suspension.

## Claims

1. A primer set for measuring the number of oral streptococci, comprising at least one forward primer comprising an oligonucleotide part of at least 15 sequential bases in a base sequence of SEQ ID NO: 1, and at least one reverse primer comprising an oligonucleotide part of at least 15 sequential bases in a base sequence of SEQ ID NO: 2

2. The primer set according to claim 1, wherein the forward primer is an oligonucleotide of the base sequence of SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, or SEQ ID NO:14, or a mixture of two or more of the oligonucleotides, and the reverse primer is an oligonucleotide of the base sequence of SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, or SEQ ID NO:26, or a mixture of two or more of the oligonucleotides.

3. A primers-probe set comprising the primer set according to claim 1 or 2, and at least one probe comprising an oligonucleotide part of at least 10 sequential bases in a base sequence of SEQ ID NO: 3 or 4.

4. The primers-probe set according to claim 3, wherein the probe is an oligonucleotide of the base sequence of SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, or SEQ ID NO:45 or a mixture of two or more of the oligonucleotides.

5. A method for measuring the number of oral streptococci by a real time PCR assay using the primer set according to claim 1 or 2.

6. A method for measuring the number of oral streptococci by a real time PCR assay using the primers-probe set according to claim 3 or 4.

7. A real time PCR kit for measuring the number of oral streptococci, comprising the primer set according to claim 1 or 2.

8. A real time PCR kit for measuring the number of oral streptococci, comprising the primers-probe set according to claim 3 or 4.


SEQUENCE LISTING

<110>  GC CORPORATION

<120>  A method for measuring the number of oral streptococci, and a PCR
primers-probe set used for the same

<130>  GCD06001

<150>  JP 2007-21423
<151>  2007-01-31

<160>  58

<170>  PatentIn version 3.1

<210>  1
<211>  30
<212>  DNA
<213>  Streptococcus mutans

<400>  1
ggttttcgga tcgtaaagct ctgttgtaag                                        30


<210>  2
<211>  28
<212>  DNA
<213>  Streptococcus mutans

<400>  2
aatccggaca acgctcggga cctacgta                                          28


<210>  3
<211>  30
<212>  DNA
<213>  Streptococcus mutans

<400>  3
taccagaaag ggacggctaa ctacgtgcca                                        30


<210>  4
<211>  30
<212>  DNA
<213>  Streptococcus mutans

<400>  4

tggcacgtag ttagccgtcc ctttctggta 30

<210> 5
<211> 24
<212> DNA
<213> Artificial

<220>
<223> primer

<400> 5
gttttcggat cgtaaagctc tgtt 24

<210> 6
<211> 23
<212> DNA
<213> Artificial

<220>
<223> primer

<400> 6
ttttcggatc gtaaagctct gtt 23

<210> 7
<211> 24
<212> DNA
<213> Artificial

<220>
<223> primer

<400> 7
ttttcggatc gtaaagctct gttg 24

<210> 8
<211> 23
<212> DNA
<213> Artificial

<220>
<223> primer

<400> 8
tttcggatcg taaagctctg ttg 23

<210>  9
<211>  22
<212>  DNA
<213>  Artificial

<220>
<223>  primer

<400>  9
ttcggatcgt aaagctctgt tg                                                22


<210>  10
<211>  23
<212>  DNA
<213>  Artificial

<220>
<223>  primer

<400>  10
ttcggatcgt aaagctctgt tgt                                               23


<210>  11
<211>  24
<212>  DNA
<213>  Artificial

<220>
<223>  primer

<400>  11
ttcggatcgt aaagctctgt tgta                                              24


<210>  12
<211>  24
<212>  DNA
<213>  Artificial

<220>
<223>  primer

<400>  12
tcggatcgta aagctctgtt gtaa                                              24


<210>  13

<211>  25
<212>  DNA
<213>  Artificial

<220>
<223>  primer

<400>  13
tcggatcgta aagctctgtt gtaag                                                25


<210>  14
<211>  24
<212>  DNA
<213>  Artificial

<220>
<223>  primer

<400>  14
cggatcgtaa agctctgttg taag                                                 24


<210>  15
<211>  16
<212>  DNA
<213>  Artificial

<220>
<223>  primer

<400>  15
tccggacaac gctcgg                                                          16


<210>  16
<211>  16
<212>  DNA
<213>  Artificial

<220>
<223>  primer

<400>  16
cggacaacgc tcggga                                                          16


<210>  17
<211>  17
<212>  DNA

<213>  Artificial

<220>
<223>  prier

<400>  17
cggacaacgc tcgggac                                                    17


<210>  18
<211>  20
<212>  DNA
<213>  Artificial

<220>
<223>  primer

<400>  18
cggacaacgc tcgggaccta                                                 20


<210>  19
<211>  17
<212>  DNA
<213>  Artificial

<220>
<223>  primer

<400>  19
ggacaacgct cgggacc                                                    17


<210>  20
<211>  18
<212>  DNA
<213>  Artificial

<220>
<223>  priemr

<400>  20
ggacaacgct cgggacct                                                   18


<210>  21
<211>  19
<212>  DNA
<213>  Artificial

<220>
<223>  primer

<400>  21
ggacaacgct cgggaccta                                                    19


<210>  22
<211>  20
<212>  DNA
<213>  Artificial

<220>
<223>  primer

<400>  22
ggacaacgct cgggacctac                                                   20


<210>  23
<211>  19
<212>  DNA
<213>  Artificial

<220>
<223>  primer

<400>  23
acaacgctcg ggacctacg                                                    19


<210>  24
<211>  18
<212>  DNA
<213>  Artificial

<220>
<223>  primer

<400>  24
caacgctcgg gacctacg                                                     18


<210>  25
<211>  19
<212>  DNA
<213>  Artificial

<220>
<223>  primer

<400> 25
caacgctcgg gacctacgt 19

<210> 26
<211> 20
<212> DNA
<213> Artificial

<220>
<223> primer

<400> 26
caacgctcgg gacctacgta 20

<210> 27
<211> 18
<212> DNA
<213> Artificial

<220>
<223> probe

<400> 27
taccagaaag ggacggct 18

<210> 28
<211> 19
<212> DNA
<213> Artificial

<220>
<223> probe

<400> 28
taccagaaag ggacggcta 19

<210> 29
<211> 29
<212> DNA
<213> Artificial

<220>
<223> probe

<400> 29

taccagaaag ggacggctaa ctacgtgcc 29

<210> 30
<211> 17
<212> DNA
<213> Artificial

<220>
<223> probe

<400> 30
accagaaagg gacggct 17

<210> 31
<211> 18
<212> DNA
<213> Artificial

<220>
<223> probe

<400> 31
accagaaagg gacggcta 18

<210> 32
<211> 28
<212> DNA
<213> Artificial

<220>
<223> probe

<400> 32
accagaaagg gacggctaac tacgtgcc 28

<210> 33
<211> 16
<212> DNA
<213> Artificial

<220>
<223> probe

<400> 33
ccagaaaggg acggct 16

<210>  34
<211>  17
<212>  DNA
<213>  Artificial

<220>
<223>  probe

<400>  34
ccagaaaggg acggcta                                                    17


<210>  35
<211>  27
<212>  DNA
<213>  Artificial

<220>
<223>  probe

<400>  35
ccagaaaggg acggctaact acgtgcc                                         27


<210>  36
<211>  17
<212>  DNA
<213>  Artificial

<220>
<223>  probe

<400>  36
cagaaaggga cggctaa                                                    17


<210>  37
<211>  27
<212>  DNA
<213>  Artificial

<220>
<223>  probe

<400>  37
cagaaaggga cggctaacta cgtgcca                                         27


<210>  38

<211>  19
<212>  DNA
<213>  Artificial

<220>
<223>  probe

<400>  38
agaaagggac ggctaacta                                                    19


<210>  39
<211>  16
<212>  DNA
<213>  Artificial

<220>
<223>  probe

<400>  39
aaagggacgg ctaact                                                       16


<210>  40
<211>  17
<212>  DNA
<213>  Artificial

<220>
<223>  probe

<400>  40
aaagggacgg ctaacta                                                      17


<210>  41
<211>  19
<212>  DNA
<213>  Artificial

<220>
<223>  probe

<400>  41
tagccgtccc tttctggta                                                    19


<210>  42
<211>  14
<212>  DNA

<213>  Artificial

<220>
<223>  probe

<400>  42
agccgtccct ttct                                                          14


<210>  43
<211>  18
<212>  DNA
<213>  Artificial

<220>
<223>  probe

<400>  43
agccgtccct ttctggta                                                      18


<210>  44
<211>  13
<212>  DNA
<213>  Artificial

<220>
<223>  probe

<400>  44
gccgtccctt tct                                                           13


<210>  45
<211>  14
<212>  DNA
<213>  Artificial

<220>
<223>  probe

<400>  45
ccgtcccttt ctgg                                                          14


<210>  46
<211>  1482
<212>  DNA
<213>  Streptococcus mutans

<400> 46
ctggctcagg acgaacgctg gcggcgtgct taatacatgc aagtggacgc aaggaaacac 60
actgtgcttg cacaccgtgt tttcttgagt cgcgaacggg tgagtaacgc gtaggtaacc 120
tgcctattag cgggggataa ctattggaaa cgatagctaa taccgcataa tattaattat 180
tgcatgataa ttgattgaaa gatgcaagcg catcactagt agatggacct gcgttgtatt 240
agctagttgg taaggtaaga gcttaccaag gcgacgatac atagccgacc tgagagggtg 300
atcggccaca ctgggactga gacacggccc agactcctac gggaggcagc agtagggaat 360
cttcggcaat ggacgaaagt ctgaccgagc aacgccgcgt gagtgaagaa ggttttcgga 420
tcgtaaagct ctgttgtaag tcaagaacgt gtgtgagagt ggaaagttca cacagtgacg 480
gtagcttacc agaaagggac ggctaactac gtgccagcag ccgcggtaat acgtaggtcc 540
cgagcgttgt ccggatttat tgggcgtaaa gggagcgcag gcggtcagga aagtctggag 600
taaaaggcta tggctcaacc atagtgtgct ctggaaactg tctgacttga gtgcagaagg 660
ggagagtgga attccatgtg tagcggtgaa atgcgtagat atatggagga acaccagtgg 720
cgaaagcggc tctctggtct gtcactgacg ctgaggctcg aaagcgtggg tagcgaacag 780
gattagatac cctggtagtc cacgccgtaa acgatgagtg ctaggtgtta ggccctttcc 840
ggggcttagt gccggagcta acgcaataag cactccgcct ggggagtacg accgcaaggt 900
tgaaactcaa aggaattgac gggggcccgc acaagcggtg gagcatgtgg tttaattcga 960
agcaacgcga agaaccttac caggtcttga catcccgatg ctattcttag agataggaag 1020
ttacttcggt acatcggaga caggtggtgc atggttgtcg tcagctcgtg tcgtgagatg 1080
ttgggttaag tcccgcaacg agcgcaaccc ttattgttag ttgccatcat taagttgggc 1140
actctagcga gactgccggt aataaaccgg aggaaggtgg ggatgacgtc aaatcatcat 1200
gccccttatg acctgggcta cacacgtgct acaatggtcg gtacaacgag ttgcgagccg 1260
gtgacggcaa gctaatctct gaaagccgat ctcagttcgg attggaggct gcaactcgcc 1320
tccatgaagt cggaatcgct agtaatcgcg gatcagcacg ccgcggtgaa tacgttcccg 1380
ggccttgtac acaccgcccg tcacaccacg agagtttgta acacccgaag tcggtgaggt 1440
aaccttttaa gggccaagcc gcctaaggtg ggatggatga tt 1482

<210> 47
<211> 22
<212> DNA
<213> Artificial

<220>
<223> primer

<400> 47
gtgtagcggt gaaatgcgta ga 22


<210> 48
<211> 22
<212> DNA
<213> Artificial

<220>
<223> primer

<400> 48
cccgtcaatt cctttgagtt tc 22


<210> 49
<211> 21
<212> DNA
<213> Artificial

<220>
<223> probe

<400> 49
caggattaga taccctggta g 21


<210> 50
<211> 22
<212> DNA
<213> Artificial

<220>
<223> primer

<400> 50
ggctacacac gtgctacaat gg 22

<210>  51
<211>  19
<212>  DNA
<213>  Artificial

<220>
<223>  primer

<400>  51
aaggcccggg aacgtattc                                                    19


<210>  52
<211>  15
<212>  DNA
<213>  Artificial

<220>
<223>  probe

<400>  52
tcgctagtaa tcgcg                                                        15


<210>  53
<211>  24
<212>  DNA
<213>  Artificial

<220>
<223>  primer

<400>  53
cgctagtaat cgcggatcag cacg                                              24


<210>  54
<211>  18
<212>  DNA
<213>  Artificial

<220>
<223>  primer

<400>  54
tgtgacgggc ggtgtgta                                                     18


<210>  55
<211>  20

<212>  DNA
<213>  Artificial

<220>
<223>  probe

<400>  55
cggtgaatac gttcccgggc                                                    20


<210>  56
<211>  25
<212>  DNA
<213>  Artificial

<220>
<223>  primer

<400>  56
gcctacagct cagagatgct attct                                              25


<210>  57
<211>  23
<212>  DNA
<213>  Artificial

<220>
<223>  primer

<400>  57
gccatacacc actcatgaat tga                                                23


<210>  58
<211>  25
<212>  DNA
<213>  Artificial

<220>
<223>  probe

<400>  58
tggaaatgac ggtcgccgtt atgaa                                              25